# EUROPEAN PATENT APPLICATION

(11) **EP 1 260 591 A1**
(43) Date of publication of application: **27.11.2002**
(21) Application number: 02253352.5
(22) Date of filing: 14.05.2002
(51) Int. Cl.: C12Q 1/48

(54) **High-efficiency assay for protein mannosyl transferases**

(30) Priority: 16.05.2001 US 291487 P
(71) Applicant: Pharmacia & Upjohn Company, Kalamazoo, MI 49007 (US)
(72) Inventor: Elhammer, Ake P., Kalamazoo, Michigan 49009 (US); Kezdy, Ferenc J., Kalamazoo, Michigan 49008 (US)
(74) Representative: Perry, Robert Edward

(57) **Abstract**

The ability of protein mannosyl transferases to catalyse the transfer of mannose from the donor sugar lipid dolichyl phosphoryl mannose to the acceptor peptide is assayed. The assay provides agents or compounds that inhibit the activity of protein mannosyl transferases.

## Description

### BACKGROUND OF THE INVENTION

### Field of the Invention

This invention relates generally to development and use of antifungal agents and more specifically to activity assay procedures for protein mannosyl transferases and uses thereof, as well as to agents or compounds identified as inhibitors of protein mannosyl transferase activity and uses thereof.

### Related Technology

Protein Mannosyl Transferases (PMTs) catalyze the transfer of mannose from the donor sugar lipid dolichyl phosphoryl mannose (Dol-P-Man) to serine and threonine residues on yeast and fungal secreted and cell surface proteins. The glycosylated products generated by this reaction are essential components of the fungal cell wall. Mutations in the PMT1 gene of *C. albicans* results in complete loss of the virulence of this organism in mice. Consequently, PMTs are important targets for anti-fungal agents.

PMT genes have been cloned for both *S. cervisiae* and *C. albicans*: S. *cervisiae* contains seven PMT genes, *C. albicans* contains two genes. Although specific functions of the different PMT sequences is unknown, the PMT enzymes are integral membrane proteins located in the endoplasmic reticulum. Analysis of the amino acid sequences of these enzymes suggest that they contain at least six membrane-spanning domains.

The availability of an effective PMT activity assay facilitates the development of PMT modulators that are useful in the development of antifungal agents. However, assays for PMT activity are complicated by the fact that both the enzyme(s) and the donor substrate (Dol-P-Man) are essentially insoluble in aqueous buffers, thereby requiring the use of comparatively harsh detergent extraction procedures (1) to solubilize the enzymes from microsomal membranes and detergent-containing buffers and (2) to keep the enzymatic activity in solution. In the cell, the hydrophobic dolichol portion of Dol-P-Man is believed to be embedded in the hydrophobic core of the endoplasmic reticulum membrane. Therefore, detergent systems are currently the only means of delivering Dol-P-Man to the PMT enzymes(s). Problems associated with the poor solubility of Dol-P-Man include inconsistent results caused by variations in the proportion of the Dol-P-Man that is in solution at any given time. Another problem associated with PMT assays is the separation of reaction product from the substrate. Published procedures all use solvent extraction to accomplish the separation. Such procedures are labor intensive and frequently generate inconsistencies in the data due to variations in, for example extraction efficiency and pipetting techniques [Strahl-Bolsinger *et al., J. Biol. Chem.*, **274:**9068-9075 (1999); Timpel *et al., J. Biol. Chem.,* **273:**20837-20846 (1998); Dotson *et al., Arch. Biochem. Biophys.,* **316:**773-779 (1995); Gentzsch *et al., FEBS Lett.*, **377:**128-130 (1995); Weston *et al., Eur. J. Biochem.*, **215:**845-849 (1993); Lorenz *et al., Eur. J. Biochem.,* **205:**1163-1167 (1992); Sharma *et al., Glycobiology*, **1:**367-373 (1991); and Strahl-Bolsinger *et al., Eur. J. Biochem.,* **196:**185-190 (1991)].

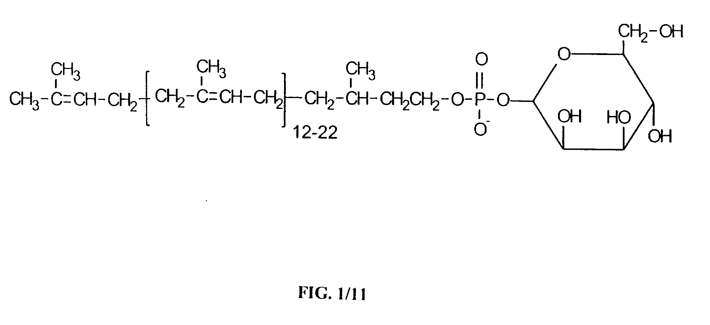

Although the PMT enzymes(s) may be kept reasonably well solubilized by the inclusion of detergents into the assay buffers, Dol-P-Man is poorly solubilized under most conditions compatible with enzymatic activity. The hydrophobic dolichol portion of the molecule, which consists of 12 to 22 polyprenol units (see Fig. 1) is too large to "fit" into most detergent micelles. Thus, there exists a need for a PMT assay procedure that overcomes the problems of the procedures currently in use, that allows for high-throughput PMT analysis, and that provides a system for screening for inhibitors of PMT activity.

### SUMMARY OF THE INVENTION

The present invention is directed to novel methods for quantifying the activity of protein mannosyl transferases comprising the steps of: contacting protein mannosyl transferase (PMT), dolichyl phosphoryl mannose, and an acceptor peptide together under conditions suitable for PMT enzymatic activity, wherein the dolichyl phosphoryl mannose is incorporated into unilamellar phospholipid vesicles; and, determining the amount glycosylated acceptor peptide produced

The present invention is also directed to methods for identifying agents that inhibit the activity of protein mannosyl transferases comprising the steps of: (a) contacting protein mannosyl transferase (PMT), dolichyl phosphoryl mannose, and an acceptor peptide together under conditions suitable for PMT enzymatic activity, in the presence of and absence of a test agent, wherein the dolichyl phosphoryl mannose is incorporated into phospholipid unilamellar vesicles; (b) measuring PMT-mediated transfer of mannose from the dolichyl phosphoryl mannose to the acceptor peptide in the presence and absence of the test agent; and (c) comparing the measurement of PMT-mediated transfer of mannose from dolichyl phosphoryl mannose to the acceptor peptide in the presence of the test agent to the measurement of PMT-mediated transfer of mannose from dolichyl phosphoryl mannose to the acceptor peptide in the absence of the test agent, wherein reduced transfer of mannose in the presence of the test agent identifies an agent that inhibits protein mannosyl transferase activity.

In another of its aspects, the present invention is directed to methods of treating fungal infection comprising the step of administering to an individual in need thereof an agent identified as an inhibitor of protein mannosyl transferases activity according to the methods for identifying such agents provided herein.

As yet a further aspect of the invention is directed to an anti-fungal agent comprising an inhibitor of protein mannosyl transferases activity identified as such according to the methods for identifying agents that inhibit the activity of protein mannosyl transferases provided herein

The present invention is also directed to a composition comprising an anti-fungal agent identified as an inhibitor of protein mannosyl transferases activity according to the methods for identifying such agents provided herein and an acceptable carrier.

In another of its aspects, the present invention is directed to a fungicidal composition comprising an inhibitor of PMT activity as identified according to the methods for identifying such agents provided herein and an acceptable carrier.

The present invention is also directed to methods of manufacturing a composition comprising an inhibitor of protein mannosyl transferase comprising the steps of: (a) contacting protein mannosyl transferase (PMT), dolichyl phosphoryl mannose, and an acceptor peptide together under conditions suitable for PMT enzymatic activity, in the presence of and absence of a test agent, wherein the dolichyl phosphoryl mannose is incorporated into unilamellar phospholipid vesicles; (b) measuring PMT-mediated transfer of mannose from the dolichyl phosphoryl mannose to the acceptor peptide in the presence and absence of the test agent; (c) comparing the measurement of PMT-mediated transfer of mannose from dolichyl phosphoryl mannose to the acceptor peptide in the presence of the test agent to the measurement of PMT-mediated transfer of mannose from dolichyl phosphoryl mannose to the acceptor peptide in the absence of the test agent, wherein reduced transfer of mannose in the presence of the test agent identifies an agent that inhibits protein mannosyl transferase activity; and (d) combining the inhibitor of PMT identified in step (c) with an acceptable carrier.

As yet a further aspect of the present invention is directed to methods of inhibiting fungal growth comprising the steps of: (a) contacting protein mannosyl transferase (PMT), dolichyl phosphoryl mannose, and an acceptor peptide together under conditions suitable for PMT enzymatic activity, in the presence of and absence of a test agent, wherein the dolichyl phosphoryl mannose is incorporated into unilamellar phospholipid vesicles; (b) measuring PMT-mediated transfer of mannose from the dolichyl phosphoryl mannose to the acceptor peptide in the presence and absence of the test agent; (c) comparing the measurement of PMT-mediated transfer of mannose from dolichyl phosphoryl mannose to the acceptor peptide in the presence of the test agent to the measurement of PMT-mediated transfer of mannose from dolichyl phosphoryl mannose to the acceptor peptide in the absence of the test agent, wherein reduced transfer of mannose in the presence of the test agent identifies an agent that inhibits protein mannosyl transferase activity; and (d) contacting a fungal organism with the test agent identified as an inhibitor of PMT in an amount effective to inhibit fungal growth.

In another of its aspects, the present invention is directed to methods for quantifying the activity of protein mannosyl transferases comprising the steps of: (a) contacting protein mannosyl transferase (PMT), dolichyl phosphoryl mannose, and an acceptor peptide together under conditions suitable for PMT enzymatic activity, wherein the dolichyl phosphoryl mannose is incorporated into unilamellar phospholipid vesicles; (b) separating the glycosylated acceptor peptide from unreacted dolichyl phosphoryl mannose via liquid chromotgraphy, wherein the chromatographic substrate is a reverse-phase matrix ; and (c) determining the amount glycosylated acceptor peptide produced.

The present invention is also directed to compositions comprising dolichyl phosphoryl mannose incorporated into a large unilamellar vesicles.

Other aspects and advantages of the invention will be apparent to those skilled in the art from a review of the following detailed description, including any drawings, as well as the claims.

### BRIEF DESCRIPTION OF THE DRAWINGS

Fig. 1 shows the chemical structure of the donor sugar lipid dolichylphosphorylmannose (Dol-P-Man), wherein PMTs catalyze the transfer of mannose from Dol-P-Man to a serine or threonine residue on secreted and cell surface proteins of fungi.

Fig. 2 is a bar graph that sets forth results of a PMT assay using detergent-solubilized Do-P-Man. Two different detergent systems were evaluated for delivery of Dol-P-Man.

Fig. 3 is a bar graph that shows results of a PMT assay using Large Unilamellar Vesicle (LUV) and detergent donor substrate delivery systems.

Fig. 4 is a bar graph that shows separation of ³H-labeled Dol-P-Man on C₁₈ cartridges.

Fig. 5 is a bar graph that sets forth the effect of LUV lipid composition on the PMT assay.

Figs. 6A and 6B are bar graphs that show the effects of different detergents on PMT assays using LUVs.

Figs. 7A and 7B are bar graphs that show that the enzyme requires a divalent cation (Fig. 7A) but that EDTA does not inhibit the enzyme under the assay conditions used (Fig. 7B).

Fig. 8. sets forth the determination of the kinetic parameters for the acceptor peptide YNPTSV.

Figs. 9 and 10 shows the separation of PMT assay reaction products on reverse-phase HPLC.

Fig. 11 shows results that indicate that PMTs retain enzymatic activity at low temperatures.

### DETAILED DESCRIPTION OF THE INVENTION

The invention described herein is a novel assay procedure for PMT activity that circumvents the problems associated with the PMT assays currently in use. The procedure utilizes a Unilamellar Phospholipid Vesicle (UPV; *e.g.,* Large unilamellar vesicles, LUVs ) delivery system for Dol-P-Man, which is capable of keeping the Dol-P-Man dispersed (if not solubilized) at all times, virtually eliminating variations in the amount of donor substrate delivered to the reaction as well as increasing the enzymatic transfer efficiency by more than ten times over the current procedures. Further, the reaction product is isolated on commercially available chromatographic columns, such as C₁₈ cartridges, rather than by the previously used solvent extraction procedures, thus decreasing the amount of labor and increasing reproducibility compared to solvent extraction with concomitant low, consistent background.

For example, the assay quantifies the amount of a label (*e.g.,* radioactivity) incorporated into an acceptor peptide, which is defined as a peptide containing an acceptor site or sites for PMTs *(e.g,* YNPTSV (SEQ ID NO: 1; see Example 4 for other exemplary peptides) from dolichyl phosphoryl [³H]-mannose (Dol-P-Man). Large unilamellar vesicles (LUVs), composed of any suitable phospholipid (such as I-palmitoyl-2-oleoyl-sn-glycero-3-phosphocholine, POPC), are used to keep the poorly soluble donor substrate Dol-P-Man in solution. In a reaction mixture conprising of 100 µg POPC LUVs, 9x10⁵ cpm (approximately 15 pmol) Dol-P-Man, 100 nmol YNPTSV and approximately 4 µg PMT-containing crude yeast microsomal lysate, the time dependence of glycosylated product formation obeys Michaelis-Menten-type kinetics throughout the course of the reaction (until exhaustion of the donor substrate). The linear initial rates of the reaction allowed calculation of an apparent Kₘ of 1.5 mM, for the acceptor peptide YNPTSV. Variations in detergent concentration (see Example 8) in the assay appear to influence transfer efficiency, possibly through interference with the LUV-based donor substrate delivery system. Thus for the highest efficiency, detergent concentration should be kept constant.

As discussed above, the glycosylated products generated by the PMT-catalyzed transfer of mannose from Dol-P-Man are essential components of the fungal cell well (noted by the fact that mutations in the PMT-1 gene in *C. albicans* resulted in a complete loss of the virulence of this organism in mice) and as such, PMTs are important targets for anti-fungal agents. Therefore, the invention is particularly useful for screening for anti-fungal agents by use of the novel PMT assay procedure.

Having generally described the invention, the same will be more readily understood by reference to the following examples, which are provided by way of illustration and are not intended as limiting.

### Example 1

### Preparation of Spheroplasts

In order to insure a supply of fungi cellular membrane material to produce PMTs, spheroplasts (protoplasts that retain some cell wall material) were produced by the following method. Specifically, *S. cervisiae* (strain BY4743) was inoculated from frozen glycerol stock into 100 ml volumes of YPD (Yeast Extract Peptone Dextrose media) medium containing yeast extract, peptone and dextrose at concentrations of 1%, 2%, and 2%, respectively. The inoculated 100 ml volumes contained in 500 ml wide mouth fermentation flasks were incubated for 30 hours at 30 °C with agitation at 225 rpm. The 30 hour seed fermentations were used at a 5% rate to inoculate the production fermentation. The production fermentation was carried out as above for 15 to 18 hours until the turbidity at 600 nm reached approximately 22 units. One hundred and twenty flasks were used to attain a 12 L fermentation volume. Harvest was by centrifugation and the cell pellets were stored without washing at -80 °C. Frozen cell pellets (43 g) were thawed and suspended in 150 ml ice-cold water. The cells were sedimented at 1500 x g for 5 minutes and the supernatant was discarded. The pellet was suspended in 45 ml and 50 mM Tris-HCl, pH 7.5, 10 mM MgCl₂. 1 M sorbitol, 30 mM DTT and incubated for 15 minutes at room temperature. The cells were subsequently sedimented at 1500 x g for 5 minutes, the supernatant was discarded and the pellet resuspended in 150 ml 50 mM Tris-HCl, pH 7.5, 10 mM MgCl₂, 1 M sorbitol, 30 mM DTT and spheroplasts were prepared as follows [procedure from Current Protocols in Molecular Biology, John Wiley & Sons, Inc. (1993)].

Specifically, the wet weight (in grams) of yeast cells in the pellet was determined by the weight increase over that of the preweighed bottle [this is approximately equal to the packed cell volume (in ml), and for all subsequent steps is considered 1 vol [one liter of BJ926 (a diploid strain) at OD₆₀₀ = 1.0 yields a packed cell volume of 2 to 3 ml]. The cells were resuspend in 2 to 4 vol ice-cold water and immediately centrifuged for 5 minutes at 1500 x *g* (SS-34 or SA-600 rotor at 3500 rpm), 4°C. The supernatant was discarded. The cells were again resuspend by adding 1 vol zymolyase buffer containing 30 mM DTT and incubated for 15 minutes at room temperature (this step facilitates subsequent zymolyase treatment and spheroplast lysis by cleaving disulfide bonds). Following incubation the cells were centrifuged for 5 minutes at 1500 x *g*, 4°C, and resuspend in 3 vol zymolyase buffer (adding 2 mg (200 U) Zymolyase 100T per ml of original packed cell volume to the resuspended cells). The resuspended cells were incubated for 40 minutes at 30°C on a shaker platform at ∼50 rpm. Following incubation, a determination was made as to whether the conversion to spheroplasts had been completed by the lysis in water technique (if spheroplasting is determined to be incomplete, incubation is continued until complete). From this point forward all procedures were performed at 4°C.

The resulting spheroplasts were centrifuged for 5 minutes at 1500 x *g*. The supernatant was carefully decanted in view of that fact that the spheroplast pellet was not as well packed as the previous cell pellets (see above). Under normal procedures the spheroplasts pellet was gently resuspended (washed) in 2 vol ice-cold zymolyase buffer and centrifuged for 5 minutes at 1500 x *g*. This step was repeated twice (for a total of three resuspensions). The resuspension/washing step removes proteases, phosphatases, and nucleases present in the zymolyase preparation. For that reason, it may be necessary to carry out additional washes.

Spheroplasts are sticky and difficult to resuspend. To facilitate resuspension, the spheroplasts were initially resuspended in a small volume with the aid of a rubber policeman. Additional buffer was then added to achieve the correct final volume.

Following washing (3x) in 50 mM Tris-HCl, pH 7.5, 10 mM MgCl₂, 1 M sorbitol, 1mM DTT the spheroplasts were (stored) frozen at -20°C.

### Example 2

### Preparation of Crude PMTs

Crude PMTs, for use in comparison experiments against the UPV system disclosed herein, were prepared by the following procedure using the spheroplasts.

A 10 ml pellet of frozen, sedimented, washed spheroplasts (produced by the method set forth in Example 1) was suspended in 3 volumes (30 ml.) 10 mM Tris-HCl, pH 7.5, 15 mM KC1, 1 mM Pefabloc, 1.5 mM MgCl₂, 1 mM DTT. The suspension was exposed to 800 psi for 20 minutes in a Parr bomb and then siphoned into a 50 mL centrifuge tube. The resulting crude membrane fraction was first centrifuged at 1600 x g for 10 minutes to remove unbroken spheroplasts and cellular debris and then supplemented with 1/10^{th} volume of 400 mM Tris-HCl, pH 7.5, 700 mM KC1, 40 mM MgCl₂, 1 mM DTT followed by sedimentation of membrane vesicles at 40,000 rpm (approximately 100000 x g) for 60 minutes. The sedimented vesicles were washed once in 50 mM Tris-HCl, pH 5, 5mM MgCl₂, 150 mM NaCl, 1 mM Pefabloc, 1mM DTT and then suspended in approximately 10 ml 50 mM Tris-HCl, pH 5, 5 mM MgCl₂. 20% glycerol, 1 mM Pefabloc. The protein concentration of the suspension was determined according to Lowry *et al.* [*J. Biol. Chem.,* **193:**265-75; 1951].

To extract PMTs, taurodeoxycholate (tDOC)and CHAPS were added to final concentrations of 0.5 and 1.2%, respectively (final concentration) and the protein concentration was adjusted to 10 mg/ml. The solution was stirred at 4 °C over night and unsolubilized material was sedimented at 40000 rpm for 60 minutes. The resulting detergent extract was used directly in PMT assays.

### Example 3

### Preparation of Dol-P-[³H]-Man LUVs

This example describes the procedure for producing Dol-P-Man (radiolabeled) incorporated into UPVs (*e.g.,* LUVs) for use in the examples *(e.g.* Example 5). 200 µl of a 10 mg/ml solution of 1-palmitoyl-2-oleoyl-sn-glycero-3-phosphocholine (POPC) obtained from Avanti Polar Lipids, Inc., together with 60 µl dolicyl phosphoryl [³H]-mannose (0.5 mCi/ml, 50 Ci/mmol), obtained from American Radiolabeled Chemicals, Inc., was evaporated to dryness under a stream of nitrogen in screw cap glass centrifuge tube. The evaporated material was dried by lyophilization for one hour after which 200 µl of 80 mM Tris-HCl, pH 7.5, 40 mM MgCl₂ was added and the tube was vortexed at full speed for 1 minute.

The semi-suspended material was shell-frozen in dry ice/acetone, thawed, revortexed for 1 minute and refrozen. The freeze/thaw-vortexing procedure was repeated once more (for a total of three times) and the resulting heterogeneous suspension of multi-lamellar vesicles was converted to LUVs by passaging 17 times through two 100 nm polycarbonate membranes mounted in a LiposoFast (Avestin, Inc.) extrusion apparatus. The resulting uniform liposome suspension was stored on ice until use (stable for, at a mi0nimum two weeks).

### Example 4

### Detergent-Based System for Determination of PMT Activity and Comparison of Effect of Different Detergent Systems on PMTs Activity

As discussed above, the donor substrate for PMTs, Dol-P-Man is essentially insoluble in aqueous buffers. Consequently, in all PMT assays described in the literature the Dol-P-Man is introduced as solubilized in detergents. Unfortunately, due to its size, Dol-P-Man fits poorly in most detergent micelles and the use of detergent systems often results in inconsistent results.

Specifically, PMT activity was assayed as outlined directly below, wherein two detergent systems were evaluated and compared for delivery of Dol-P-Man: Buffer I (20 mM Tris-HCl, pH 7.5, 10 mM MgCl₂, 0.2% Triton X-100, 0.15% CHAPS, 0.15% taurodeoxycholate) and Buffer IV (10 mM Tris-HCl, pH 7.5, 5 mM MgCl₂, 0.1% Triton X-100, 0.075% CHAPS, and 0.2% taurodeoxycholate).

The acceptor peptide used for all experiments (YNPTSV; SEQ ID NO: 1) was synthesized as outlined previously [Elhammer *et al., J. Biol. Chem.*, 268:10029-38; 1993] Other peptides containing an acceptor site or sites for PMTs that may be used to practice the present invention include (but are not limited to) YPTAV (SEQ ID NO: 2); PTV (SEQ ID NO: 3); PYTV (SEQ ID NO: 4); YPTAV (SEQ ID NO: 5); YNPTAV (SEQ ID NO: 6); YNLTSV (SEQ ID NO: 7); YNPASV (SEQ ID NO: 8); YNLTSV (SEQ ID NO: 9); YDLTSV (SEQ ID NO: 10); YQLTSV (SEQ ID NO: 11); PPASTSAPG (SEQ ID NO: 12); PPDAATAAPL (SEQ ID NO: 13); PPDAASAAPL (SEQ ID NO: 14); PPASTSAPG (SEQ ID NO: 15); PPASSSAPG (SEQ ID NO: 16); and VVPTVVPG (SEQ ID NO: 17).

Assays using detergent solubilized donor substrate (instead of LUVs) were carried out as described by Strahl-Bolsinger and Tanner [*Eur. J. Biochem.*, **196**:185-90; 1991] with some modifications. Specifically assays were carried out in 20 mM Tris-HCL, pH 7.5, 10 mM MgCl₂, 0.2% Triton X-100, 0.15 % taurodeoxycholate, 0.15% Chaps, containing 0.75µCi Dol-P-Man (approx. 15 pmol), 2.5 mM of the acceptor peptide YNPTSV and approx. 7 µg of crude enzyme.

Assay reaction products were isolated on cartridges using a reverse-phase matrix (C₁₈. 1 cc/100 mg Bond Elut; although other reverse-phase matrixes may be used, *e.g,* C₆, C₁₂) obtained from Varian, [Homa *et al. Prot. Exp. Purif.,* **6:**141-48; 1995] isolation of reaction product by solvent extraction was carried out as described by Strahl-Bolsinger and Tanner [*Eur. J. Biochem.,* **196:**185-90; 1991].

Although there are no methodological differences (*e.g.,* volumes, solvents used) between the C₁₈ procedure used here in and that used by Homa *et al.* [*Prot. Exp. Purif.*, **6:**141-48; 1995], apart from type and volume of the sample loaded, there are differences in how the cartridges work for the two assays. In the Homa *et al*. assay, the unreacted, radioactive donor substrate was washed out of the column in the washing step while in the PMT assay disclosed herein the radioactive donor substrate remains bound to the cartridge (even during the elution of the product). Essentially, it is never eluted but discarded with the used cartridge.

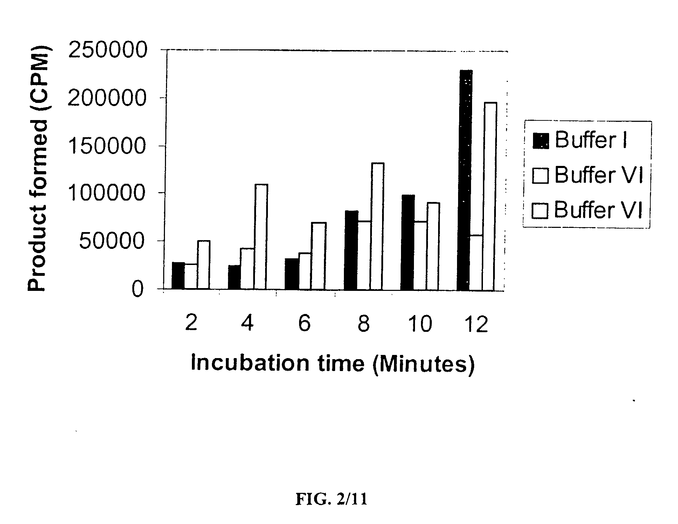

Results from the comparison of the detergent systems, which are shown in Fig. 2 [three individual experiments were conducted (Buffer I, Buffer VI, and Buffer VI) to illustrate the variability in the data generated in this type of system], clearly illustrate that although enzymatic activity is easily detected, linearity with time and/or substrate concentration is often poor. The results also indicate that detergent type and concentration thereof may have a significant impact on the product recovery in the assay. A likely explanation for this is variations in the proportion donor substrate, *i.e.* Dol-P-Man, that is actually in solution (at any given time) in the individual assay samples. It has been frequently observed that some samples (in the same assay) remained completely clear while others turned opalescent, indicating that in the latter samples (at least a portion of) the Dol-P-Man was coming out of solution. Several different detergents and detergent combinations were evaluated in an effort to remedy this problem, but none of them were capable of consistently maintaining Dol-P-Man in solution or of generating reproducible assay data.

### Example 5

### Comparison of PMT Assay Using LUVs Versus Detergent Donor Substrate Systems and Determination of Kinetic Parameters of Acceptor Peptide

To resolve the problems noted above (Example 4) an assay was developed utilizing UPVs rather than harsh detergents, to keep the Dol-P-Man in solution. Although exemplified by use of LUVs other types of Unilamellar Vesicles, *i.e.*, both larger and smaller unilamellar vesicles may be used to practice the invention. LUVs are defined as approximately 500 Å in size, while Small Unilamellar Vesicles (SUVs) are defined as being smaller than 250 Å. The POPC membranes of the LUVs provide an environment similar in dimensions and hydrophobicity to the microsomal membranes that harbor the Dol-P-Man in the cell. Incorporation of Dol-P-Man in 100 nM LUVs results in a stable semi-transparent "solution" that is quite homogeneous and stable for at least two weeks.

PMT activity was assayed using either a detergent-based delivery system for Dol-P-Man or LUVs. Methods for the detergent-based delivery system were as set forth above in Example 4 (specifically using Buffer 1). LUVs were produced by the method set forth in Example 3.

The assays using the UPV method contained 20 mM Tris-HCl, pH 7.5, 10 mM MgCl₂, 2.5 mM of the peptide, YNPTSV, 1.5 µl Dol-P-[³H]-Man (approximately 0.9 x 10⁶ cpm) "solubilized" in 100 µg POPC LUV's and 8 µg of crude enzyme (see Example 2) in a final volume of 40 µl. The assay samples were incubated for 2-30 minutes (varied per individual experiments) at 20 °C and at the end of the incubation the reaction was stopped by the addition of 10 µl 50 mM CuSO₄ or 1 ml 0.1 % TFA.

Assay reaction products were isolated on C₁₈ cartridges (1 cc/100 mg Bond Elut) obtained from Varian [Homa *et al. Prot. Exp. Purif.*, **6:**141-48; 1995]. Isolation of reaction product by solvent extraction was carried out as described by Strahl-Bolsinger and Tanner [*Eur. J. Biochem.,* **196:**185-90; 1991].

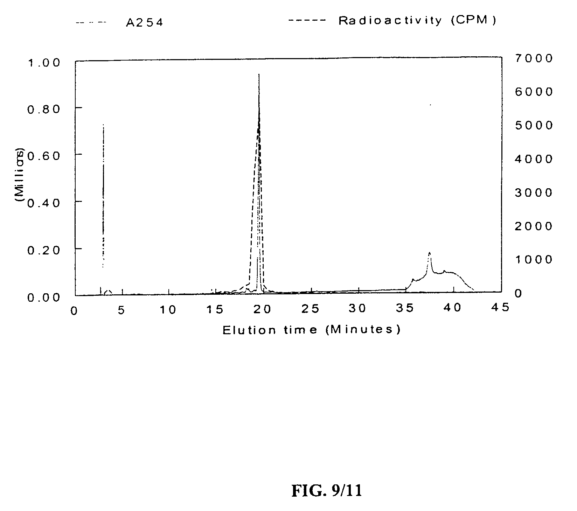

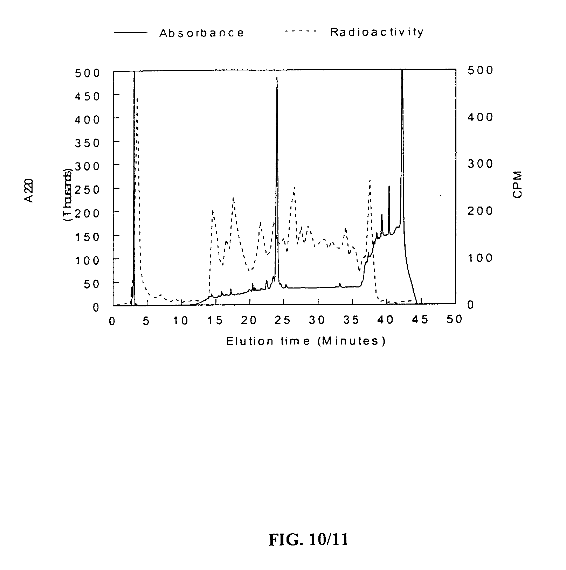

As stated above in Example 4, all the assays for which data are shown herein were the result of use of C₁₈ cartridges (although the product characterization data set forth in Figs. 9 and 10 were the result of the use of reverse-phase HPLC). While there are no methodological differences *(e.g.,* volumes, solvents used) between the C₁₈ procedure used here in and that used by Homa *et al.* [*Prot. Exp. Purif.,* **6:**141-48; 1995], apart from type and volume of the sample loaded, there are differences in how the cartridges work for the two assays. In the Homa *et al*. assay, the unreacted, radioactive donor substrate was washed out of the column in the washing step while in the PMT assay disclosed herein the radioactive donor substrate remains bound to the cartridge (even during the elution of the product). Essentially, it is never eluted but discarded with the used cartridge.

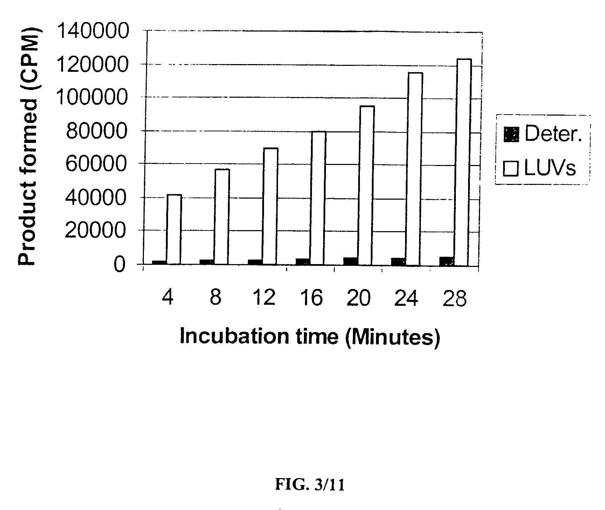

Results, which are set forth in Fig. 3, indicated that as compared to the current systems in use (detergent-based), the UPV-based assay system is both reproducible and linear. Further, the UPV donor substrate delivery system also increases assay efficiency more than ten times, compared to a detergent based assay, which results in shorter assay times and a much improved signal-to-noise ratio. Also, when comparing the results of Fig. 2 to the results of Fig.3, it is important to note that there was a five-times higher specific activity enzyme preparation added to the experiment reflected by Fig. 2, while a lower specific activity preparation of Dol-P-Man was used for the experiment reflected by the data of Fig. 3

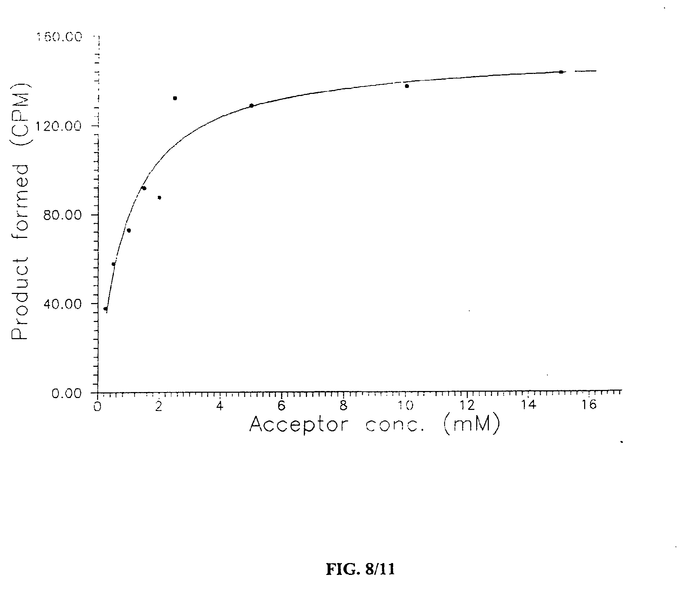

The kinetic parameters for the standard acceptor peptide, YNPTSV, were determined with the LUV-based assay (Fig. 8) set forth directly above. Specifically, the apparent Kₘ (using crude enzyme preparation) was determined to be approximately 1 mM and Vₘₐₓ was determined to be approximately 7.25 pmol min.⁻¹ mg protein⁻¹. The Kₘ value is similar to the 3.3 mM previously determined in a detergent based assay [Strahl-Bolsiner and Tanner, *Eur. J. Biochem.,* **196:**185-190; 1991] and also similar to the (*in vitro*) Kₘ values for essentially all evaluated acceptors for the mammalian polypeptide N-acetylgalactosaminyltransferases, another family of polypeptide O-glycosylating enzymes [*e.g.* Elhammer *et al.*, *Glycoconj. J.,* **16:**171-80, 1999; Wragg *et al., J. Biol. Chem.,* **270:**16947-54, 1995; and Wang *et al., J. Biol. Chem.,* **267:**12709-16, 1992]. This suggests that low mM Km values may be typical for polypeptide glycosylating enzymes in assays using small synthetic acceptor peptides. The *in vivo* Kₘ values of the *in situ* acceptors for all these enzymes remain unknown.

Overall the kinetic data provided herein does show that the enzyme obeys Michaelis-Menten type kinetics, which is of particular value in systems where crude enzyme preparations are used and where unwanted reactions/processes may influence the data. Moreover, the kinetic parameters obtained suggest that the assay indeed measures the real, or at least a close mimic, of the real transfer reaction: The numbers obtained are very close to those obtained for other polypeptide O-glycosylating enzymes assays with homogeneous enzyme preparations.

### Example 6

### Separation of ³H-labeled Dol-P-Man on C₁₈ Cartridges and Separation of Assay Reaction Products on Reverse-Phase HPLC

A significant complication associated with PMT assays has been isolation of the reaction product. Published protocols have accomplished this by solvent extraction, which results in a time-consuming complicated procedure that is poorly suited for assays involving large numbers of samples *(e.g.* Strahl-Bolsinger and Tanner, *Eur. J. Biochem.*, **196:**185-90; 1991 and Dotson *et al., Arch. Biochem. Biophys.*, **316:**773-79; 1995). The procedure involves phase-separations by centrifugation as well as several pipetting steps. Volumes and solvent composition chosen for a particular a extraction, in combination with variations in the composition of the samples, may effect the efficacy of the extraction of a sample. Further, the procedure involves the manual separation (via pipette) of one phase floating on top of another (usually in a micro tube), without disturbing the boundary layer between the two distinct layers. Such techniques lend themselves to a large number of errors.

In an effort to create a more robust and less complicated assay procedure, we investigated whether C₁₈ cartridges could be used (in place of solvent extraction) to separate the glycosylated peptide product from Dol-P-Man. Once immobilized on the C₁₈ matrix the peptide reaction product can be washed extensively (to remove contaminating radioactivity) and then eluted with acetonitrile.

While a similar approach has been used successfully to isolate the reaction products from other polypeptide glycosylating enzymes [Homa *et al., Prot. Exp. Purif.*, **6:**141-48; 1995 and O'Connel and Tabak, *Anal. Biochem.,* **210:**423-25; 1993], those enzymes all utilize nucleotide sugars, *i.e.,* hydrophilic donor substrates, that do not interact with the C₁₈ matrix, and that are easily removed by washing the cartridge. The donor substrate for all PMTs is Dol-P-Man, a molecule which, by contrast, is very hydrophobic and which one would predict would interact, at least significantly, with the matrix.

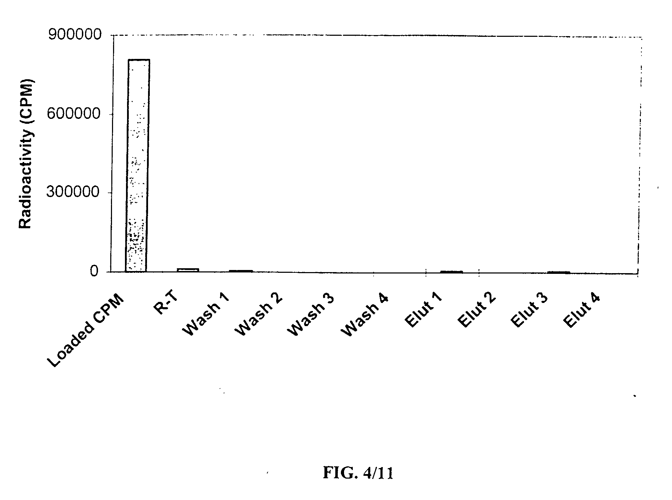

To investigate how strongly Dol-P-Man interacts with the C₁₈ matrix we first chromatographed the [³H]-labeled Dol-P-Man molecule (by itself) on a cartridge. Specifically, 803,000 cpm of Dol-P[³H]-Man in one ml of 0.1% trifluoroacetic acid (TFA) was loaded on a 100 cc C18 cartridge. The run-through (R-T) fraction (1ml) and four 1 ml was fractions were collected. The cartridge was then eluted with 2 x 1 ml 35 % acetonitrile (Elut 1 and Elut 2), followed by 2 x 1 ml 100% acetonitrile (Elut 3) and Elut 4). All fractions were analyzed for amount of radioactivity and are shown in Fig. 4. Results indicate that the radioactive molecule bound quite tightly. Virtually none of the loaded radioactivity was eluted by the 35% acetonitrile solution used for elution of acceptor peptides. Further, even 100% acetonitrile failed to elute significant amounts of the radioactive substrate. These results suggested that C₁₈ cartridges could indeed be used for separation of the reaction products from PMT assays and that there were two possible manners in which to accomplish the separation procedure.

In the first approach, the reaction sample is loaded on the cartridge in acidified water (which would result in both the reaction product and unreacted Dol-P-Man binding to the matrix); the unbound, water-soluble radioactive material is washed out; and then the glycosylated peptide is eluted with 35% acetonitrile, all resulting in unreacted Dol-P-Man remaining bound to the cartridge. With respect to the second approach, the sample is loaded in 35% acetonitrile directly onto the column and the column run-through are collected, which then will contain the glycosylated acceptor peptide, and any other radioactive species, except Dol-P-Man. Those of skill in the art will readily appreciate that other solvents, *i.e.,* methanol, isopropanol, and/or, other higher alcohol may be used in the practice of the invention.

Both approaches were evaluated and the results were similar with the exception of a slightly higher background when using this second approach. The background was slightly higher with the second approach in view of the fact that omission of the wash step would allow any hydrophilic and moderately hydrophobic contaminants to elute with the product. However, the second procedure is considerably simplified and would lend itself as a legitimate method of choice for high-through put screening applications, since the signal-to-noise ratio of the background were still acceptably high.

To verify that the radioactivity trapped on the C₁₈ cartridges (and quantified as a measure of the amount of reaction product formed) actually represented only the glycosylated acceptor peptide, a portion of the eluate from the cartridges was analyzed on reverse-phase HPLC. The chromatogram shown in Fig. 9 contains only one major peak, with a retention time of approximately 19.5 minutes. This is the exact retention time of the synthetic acceptor peptide used in the assay. Moreover, a plot of the radioactivity in fractions collected from the column eluate (dashed line) revealed that approximately 95% of the radioactivity loaded on the column eluted in a peak with a retention time almost identical to that of the acceptor peptide. The slight shift (of the radioactivity peak) towards an earlier retention time is consistent with the expected increase in hydrophilicity generated by the addition of a monosaccharide to the peptide. Taken together these observations strongly suggest that glycosylated YNPTSV is essentially the only product formed and measured by the UPV assay, under standard assay conditions, *i.e.* dilute enzyme (< 10 µg) and short incubation times (<20 minutes). However, if high enzyme concentrations (50 µg) and prolonged incubation times (30-60 minutes) are used, additional products may be formed

Further, with respect to Fig. 9, the peak at 2.5 minutes is the solvent front, which will carry through any hydrophilic radioactive contaminants in the Dol-P-Man preparation. The peak eluting at 35-45 minutes represents endogenous polypeptides (present in the crude enzyme preparation) glycosylated during the assay. Analysis of structures released from the reaction products by mild alkaline sodium borohydride treatment (which specifically cleaves O-linked glycoconjugates) suggest that this radioactivity probably is associated with N-glycans, labeled by Dol-P-Man mediated incorporation of mannose into (N-linked) trimannosyl core structures

Fig. 10 shows separation of the reaction products from a 45 minute assay containing 40 µg enzyme and a non-functional acceptor peptide (containing no hydroxy amino acid). An array of low level radioactivity peaks are clearly discernable. These probably represent various low-level or low-efficiency acceptors present in the crude enzyme preparation used in the assay. They may also represent incorporation into N-linked glycans via the (partially) Dol-P-Man mediated biosynthesis of the N-linked oligosaccharide high-mannose precursor (Kornfeld and Kornfeld, *Ann. Rev. Biochem.,* **210:**423-25, 1985). As demonstrated by the experiment shown in Fig. 9, this is not a problem under normal assay conditions when using a high-efficiency acceptor but it can be significant in experiments aimed at determining the real limits of acceptor specificity and efficiency. Clearly a purified enzyme (recombinant or native) would be a considerable asset.

### Example 7

### Effect of UPV Lipid Composition

Phospholipids other than POPC may be used in the practice of the present invention, *e.g.,* other naturally occurring phosphatidylcholines (*i.e.,* phosphatidylcholines with fatty acid different than those of POPC), phosphatidylserine, phosphatidylethanolamine, and/or phosphatidylinositol. As discussed above, the LUVs promote solubilization of the Dol-P-Man by providing a hydrophobic environment similar to that of an endoplasmic reticulum membrane. Still, the LUVs used in the experiment shown in Example 5 (Fig. 3) were composed of POPC only. The composition of endoplasmic reticulum membranes is much more complex. To investigate whether altering the composition of the LUVs to more closely resemble that of an endoplasmic reticulum membrane could yield further enhancement of the enzymatic activity in the assay, Dol-P-Man LUVs were prepared (by the methods set forth in Example 3) that contained 75 mol% POPC, 5 mol% phosphatidyl ethanolamine (PE) and 20 mol% cholesterol, *i.e.* very similar to the percentages of the major lipid components of the endoplasmic reticulum membrane.

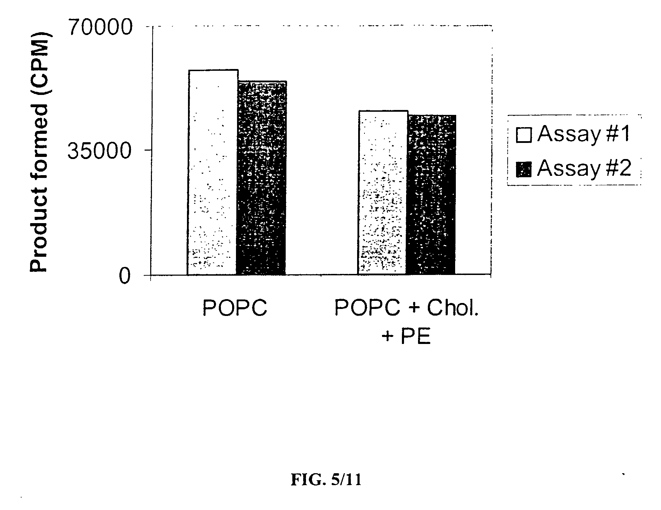

PMT activity was assayed as set forth in the methods of Example 5. Results (see Fig. 5) indicated that the increased complexity did not result in any increase in transfer efficiency.

### Example 8

### Effect of Detergents and Divalent Cation Dependence

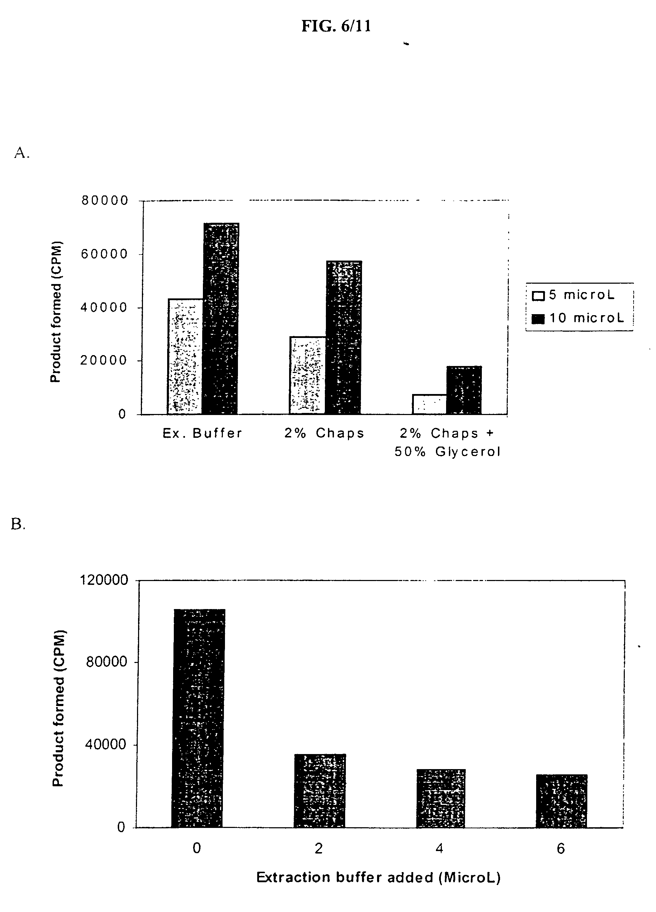

As part of the characterization of the UPV PMT assay, other parameters such as the influence of detergents and ion dependence were also investigated. PMT assays (using LUVs as produced by the method in Example 3) were conducted by the methods of Example 5. Assays were performed at two concentrations of enzymes (5 and 10 µl). Buffers containing only CHAPS or CHAPS + 50% glycerol were substituted for the standard Extraction Buffer used to solubilize the ezyme (see Fig 6A). The effect of increased detergent concentration was investigated by the addition of increasing amounts of Extraction Buffer (see Fig. 6B).

As noted in Fig. 6A, substitution of a zwitter-ionic detergent, such as CHAPS, for the taurodeoxycholate (tDOC) in the enzyme extraction buffer had a marked deleterious effect on transfer. In order to maintain the increased efficiency noted with the assay disclosed herein, the stronger solubilizing (and denaturing) capabilities of taurodeoxycholate are required to prohibit the PMT enzymes from falling out of solution. With respect to Fig. 6B, it is important to note that amount of detergents in the assay should range from 0.05% CHAPS/0.01% tDOC to 0.18% CHAPS/0.075% tDOC (final detergent concentration). A preferred amount of detergent for use in the present assay is 0.12% CHAPS/0.05% tDOC. It is interesting to note that the assay appears to be quite sensitive to glycerol, presumably because of the increase in viscosity. Further, other detergents or mixtures of detergents that may be used to practice the present invention include CHAPSO / dexoycholate, octylglucoside, Triton, Brij, and/or Lubrol.

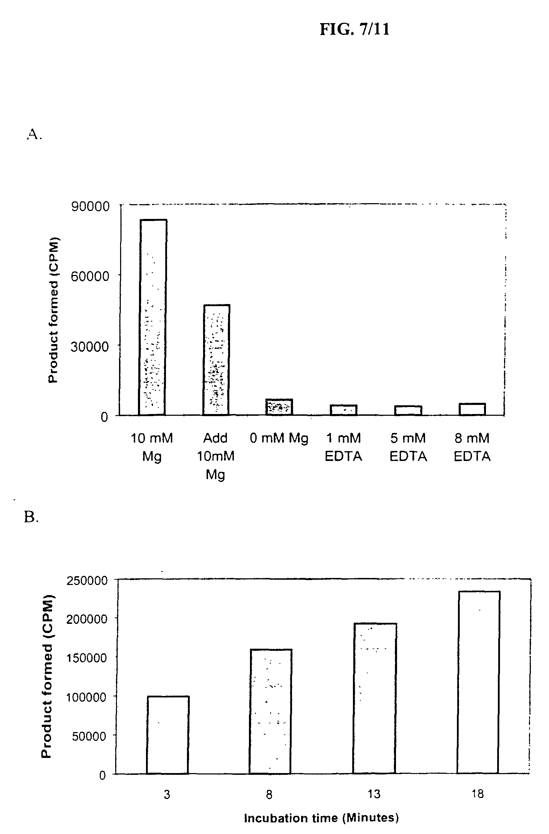

Experiments were also conducted (as described immediately above) to determine whether PMTs are ion dependent. The control assay (10 mM Mg) contained LUVs made in buffer containing 10 mM MgCl₂. All other assays contained LUVs prepared in buffer without MgCl₂. Fig. 7A sets forth PMT activity in the absence of Mg²⁺ , in the presence of added MgCl₂, and in the presence of added EDTA. The results indicate that the enzyme requires a divalent cation such as Mg²⁺. However, in contrast to many other glycosyltransferases, the divalent cation chelator EDTA appears not to inhibit the enzyme, at least not under the conditions used in the assay (Fig. 7B); two other chelators, EGTA and orthophenanthroline were equally ineffective (data not included). Thus, PMT's must have a much stronger affinity for divalent cations than, for instance, several of the glycosyltransferases in the mammalian Golgi complex [Beyer *et al., Adv. Enzymol. Relat. Areas Mol. Biol.,* **52:**23-175; 1981].

### Example 9

### Effect of Low Temperatures on PMT Enzymatic Activity

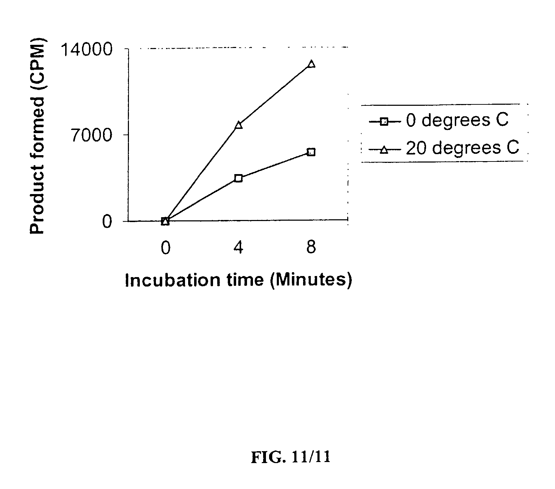

Another variable investigated was the temperature dependence of the PMT reaction using the UPV-based method. Methods were as described in Examples 3 and 5. Results indicated that PMT enzymes, at least *in vitro* and when using the UPV-based substrate, were comparatively insensitive to variations in temperature. In fact, they appeared to retain over 40% of their activity even at 0 °C (Fig. 11). Thus, in contrast to several other glycosyltransferaseses, the reaction is not terminated, not even essentially so, by placing the assay samples on ice. Thus, alternative means should be employed to bring an abrupt termination to the UPV-based PMT assay. For example, a drastic drop in pH or introduction of a toxic ion such as Cu²⁺ are preferred methods.

### Example 10

### Determination of Inhibitors of PMT Activity /Screening for Anti-Fungal Agents

As discussed above, the glycosylated products generated by the PMT-catalyzed transfer of mannose from Dol-P-Man are essential components of the fungal cell wall (noted by the fact that mutations in the PMT-1 gene in *C. albicans* resulted in a complete loss of the virulence of this organism in mice) and as such, PMTs are important targets for anti-fungal agents and compounds. Therefore, the invention is particularly useful for screening for anti-fungal agents and compounds by use of the novel PMT assay procedure.

For example, identification of inhibitors of PMT activity may be determined by the following method. Specifically, PMT activity is assayed via the UPV-based system (Example 5) either in the presence or absence of putative inhibitor. Results of the PMT-mediated transfer of mannose from Dol-P-Man to an acceptor peptide in the presence of the putative inhibitor are then compared to results obtained from the PMT-mediated transfer of mannose from Dol-P-Man to an acceptor peptide in the absence of the putative inhibitor. Comparison of these results, for example, may include Lineweaver-Burk analysis, Dixon analysis, Eisenthal &Cornish-Bowden analysis, Eadie-Scatchard anaylsis, and/or Hanes-Woolf analysis [Segel, I.H., *Enzyme Kinetics;* 1975; John Wiley & Sons, Inc.; New York, NY]. Agents considered to have anti-fungal activity are those identified as capable of inhibiting PMT activity.

Inhibitors of PMT activity may also be determined by administration of the putative inhibitor to whole cells, wherein the impact of the treatment on the glycoconjugates expressed by the cells is analyzed. More specifically, an inhibitor is predicted to block conjugation of O-linked oligosaccharides to secreted and cell surface glycoproteins. This can easily be assessed by isolating (*e.g.*, via immunoprecipitation) known O-linked oligosaccharide-containing glycoproteins from the treated cells and then quantifying the amount of O-linked oligosaccharides present on them (as compared to the same molecules isolated from untreated cells).

A further method for detection would be to again treat whole cells with the inhibitor and then a lectin or an antibody to monitor changes in the expression O-mannosyl-type carbohydrates on the cell surface.

Inhibitors may also be detected by isolating fungal cell membranes or total secreted proteins from inhibitor-treated cells and then conduct an amino acid compositional analysis on the entire mixture and compare that with the same analysis on untreated cells. Since glycosylated amino acids are not detected in the analysis, inhibition of PMTs should produce an increase in the proportion of serine and threonine, in relation to all other amino acids.

### Example 11

### Pharmaceutical Formulations

An anti-fungal agent or compound identified by the novel assay herein may be administered to an animal or a patient in need, by itself, or in pharmaceutical compositions where it is mixed with suitable carriers or excipient(s) at doses to treat or ameliorate a fungal infection. Such a composition may optionally contain (in addition to anti-fungal agent or other active ingredient and a carrier) diluents, fillers, salts, buffers, stabilizers, solubilizers, and other materials well known in the art. The term "pharmaceutically acceptable" means a non-toxic material that does not interfere with the effectiveness of the biological activity of the active ingredient(s). The characteristics of the carrier will depend on the route of administration.

The pharmaceutical composition may further contain other agents which either enhance the activity of the anti-fungal agents or other active ingredients identified by the novel assay of the present invention or compliment its activity or use in treatment. Such additional factors and/or agents may be included in the pharmaceutical composition to produce a synergistic effect with anti-fungal agents or other active ingredients identified by the novel assay of the present invention, or to minimize side effects.

Pharmaceutical compositions for use in accordance with the present invention thus may be formulated in a conventional manner using one or more physiologically acceptable carriers comprising excipients and auxiliaries which facilitate processing of the active compounds into preparations which can be used pharmaceutically. These pharmaceutical compositions may be manufactured in a manner that is itself known, *e.g.*, by means of conventional mixing, dissolving, granulating, dragee-making, levigating, emulsifying, encapsulating, entrapping or lyophilizing processes. Proper formulation is dependent upon the route of administration chosen. When a therapeutically effective amount of anti-fungal agents or other active ingredients identified by the novel assay of the present invention is administered orally, anti-fungal agents or other active ingredients identified by the novel assay of the present invention will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95%anti-fungal agents or other active ingredients identified by the novel assay of the present invention, and preferably from about 25 to 90% anti-fungal agents or other active ingredients identified by the novel assay of the present invention. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of anti-fungal agents or other active ingredients identified by the novel assay of the present invention, and preferably from about 1 to 50% anti-fungal agents or other active ingredients identified by the novel assay of the present invention.

When a therapeutically effective amount of anti-fungal agents or other active ingredients identified by the novel assay of the present invention is administered by intravenous, cutaneous or subcutaneous injection, anti-fungal agents or other active ingredients identified by the novel assay of the present invention will be in the form of a pyrogen-free, parenterally acceptable aqueous solution. The preparation of such parenterally acceptable anti-fungal agents or other active ingredients identified by the novel assay of the present invention solutions, having due regard to pH, isotonicity, stability, and the like, is within the skill in the art. A preferred pharmaceutical composition for intravenous, cutaneous, or subcutaneous injection should contain, in addition to anti-fungal agents or other active ingredients identified by the novel assay of the present invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art. For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration. For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.,* gelatin for use in an inhaler or insufflator may be formulated containing a powder mix of the compound and a suitable powder base such as lactose or starch. The compounds may be formulated for parenteral administration by injection, *e.g.*, by bolus injection or continuous infusion. Formulations for injection may be presented in unit dosage form, *e.g.,* in ampules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions. Alternatively, the active ingredient may be in powder form for constitution with a suitable vehicle, *e.g.,* sterile pyrogen-free water, before use.

The compounds may also be formulated in rectal compositions such as suppositories or retention enemas, *e.g.,* containing conventional suppository bases such as cocoa butter or other glycerides. In addition to the formulations described previously, the compounds may also be formulated as a depot preparation.. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e.g. polyvinyl pyrrolidone; and other sugars or polysaccharides may substitute for dextrose. Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various of sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for anti-fungal agents or other active ingredients identified by the novel assay of the present invention ingredient stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols. Many of the active ingredients of the invention may be provided as salts with pharmaceutically compatible counterions. Such pharmaceutically acceptable base addition salts are those salts which retain the biological effectiveness and properties of the free acids and which are obtained by reaction with inorganic or organic bases such as sodium hydroxide, magnesium hydroxide, ammonia, trialkylamine, dialkylamine, monoalkylamine, dibasic amino acids, sodium acetate, potassium benzoate, triethanol amine and the like.

The pharmaceutical composition of the invention may be in the form of a complex of the anti-fungal agents or other active ingredients identified by the novel assay of the present invention along with protein or peptide antigens. The protein and/or peptide antigen will deliver a stimulatory signal to both B and T lymphocytes. B lymphocytes will respond to antigen through their surface immunoglobulin receptor. T lymphocytes will respond to antigen through the T cell receptor (TCR) following presentation of the antigen by MHC proteins. MHC and structurally related proteins including those encoded by class I and class II MHC genes on host cells will serve to present the peptide antigen(s) to T lymphocytes. The antigen components could also be supplied as purified MHC-peptide complexes alone or with co-stimulatory molecules that can directly signal T cells. Alternatively antibodies able to bind surface immunoglobulin and other molecules on B cells as well as antibodies able to bind the TCR and other molecules on T cells can be combined with the pharmaceutical composition of the invention.

The pharmaceutical composition of the invention may be in the form of a liposome in which anti-fungal agents or other active ingredients identified by the novel assay of the present invention is combined, in addition to other pharmaceutically acceptable carriers, with amphipathic agents such as lipids which exist in aggregated form as micelles, insoluble monolayers, liquid crystals, or lamellar layers in aqueous solution. Suitable lipids for liposomal formulation include, without limitation, monoglycerides, diglycerides, sulfatides, lysolecithins, phospholipids, saponin, bile acids, and the like. Preparation of such liposomal formulations is within the level of skill in the art, as disclosed, for example, in U.S. Pat. Nos. 4,235,871; 4,501,728; 4,837,028; and 4,737,323, all of which are incorporated herein by reference.

### Example 12

### Treatment of Fungal Infections

Another embodiment of the invention is the administration of an effective amount of the antifungal agent or compounds identified by the novel assay disclosed herein to individuals affected by a fungal infection. While the mode of administration is not particularly important, parenteral administration is preferred. An exemplary mode of administration is to deliver an intravenous bolus. The dosage of the polypeptide or composition of the invention will normally be determined by the prescribing physician. It is to be expected that the dosage will vary according to the age, weight, condition and response of the individual patient. Typically, the amount of anti-fungal agents or other active ingredients identified by the novel assay of the present invention administered per dose will be in the range of about 0.1 to 25 mg/kg of body weight, with the preferred dose being about 0.1 to 10 mg/kg of patient body weight. For parenteral administration, the anti-fungal agent or agents or other active ingredients of the invention will be formulated in an injectable form combined with a pharmaceutically acceptable parenteral vehicle. Such vehicles are well known in the art and examples include water, saline, Ringer's solution, dextrose solution, and solutions consisting of small amounts of the human serum albumin (see also Example 11). The vehicle may contain minor amounts of additives that maintain the isotonicity and stability of the polypeptide or other active ingredient. The preparation of such solutions is within the skill of the art

Techniques for formulation and administration of the compounds of the instant application may be found in "Remington's Pharmaceutical Sciences," Mack Publishing Co., Easton, PA, latest edition. A therapeutically effective dose refers to that amount of the compound sufficient to result in amelioration of symptoms, e.g., treatment, healing, prevention or amelioration of the relevant medical condition, or an increase in rate of treatment, healing, prevention or amelioration of such conditions. When applied to an individual active ingredient, administered alone, a therapeutically effective dose refers to that ingredient alone. When applied to a combination, a therapeutically effective dose refers to combined amounts of the active ingredients that result in the therapeutic effect, whether administered in combination, serially or simultaneously.

In practicing the method of treatment or use of the agents identified by the present invention, a therapeutically effective amount of anti-fungal or other active ingredient identified by the novel assay of the present invention is administered to a mammal having a condition to be treated. Anti-fungal agents or other active ingredients identified by the novel assay of the present invention may be administered in accordance with the method of the invention either alone or in combination with other therapies such as anti-inflammatory agents. When coadministered with one or more anti-inflammatory agents, anti-fungal agents or other active ingredients identified by the novel assay of the present invention may be administered either simultaneously with anti-inflammatory agents, or sequentially. If administered sequentially, the attending physician will decide on the appropriate sequence of administering anti-fungal agents or other active ingredients identified by the novel assay of the present invention in combination with anti-inflammatory agents.

Suitable routes of administration may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. Administration of anti-fungal agents or other active ingredients identified by the novel assay of the present invention used in the pharmaceutical composition or to practice the method of the present invention can be carried out in a variety of conventional ways, such as oral ingestion, inhalation, topical application or cutaneous, subcutaneous, intraperitoneal, parenteral or intravenous injection.

Alternately, one may administer the compound in a local rather than systemic manner, for example, via injection of the compound directly into the effected area, often in a depot or sustained release formulation. Furthermore, one may administer the drug in a targeted drug delivery system, for example, in a liposome coated with a specific antibody. The liposomes will be targeted to and taken up selectively by the afflicted tissue.

The amount of anti-fungal agents or other active ingredients identified by the novel assay of the present invention in the pharmaceutical composition of the present invention will depend upon the nature and severity of the condition being treated, and on the nature of prior treatments which the patient has undergone. Ultimately, the attending physician will decide the amount of anti-fungal agents or other active ingredients identified by the novel assay of the present invention with which to treat each individual patient. Initially, the attending physician will administer low doses of anti-fungal agents or other active ingredients identified by the novel assay of the present invention and observe the patient's response. Larger doses of anti-fungal agents or other active ingredients identified by the novel assay of the present invention may be administered until the optimal therapeutic effect is obtained for the patient, and at that point the dosage is not increased further. It is contemplated that the various pharmaceutical compositions used to practice the method of the present invention should contain about 0.01 µg to about 100 mg (preferably about 0.1 µg to about 10 mg, more preferably about 0.1 µg to about 1 mg) of anti-fungal agents or other active ingredients identified by the novel assay of the present invention per kg body weight. The therapeutic compositions are also presently valuable for veterinary applications. Particularly domestic animals and thoroughbred horses, in addition to humans, are desired patients for such treatment with anti-fungal agents or other active ingredients identified by the novel assay of the present invention.

Pharmaceutical compositions suitable for use in the present invention include compositions wherein the active ingredients are contained in an effective amount to achieve its intended purpose. Determination of the effective amount is well within the capability of those skilled in the art, especially in light of the detailed disclosure provided herein. For any compound used in the method of the invention, the therapeutically effective dose can be estimated initially from cell culture assays. For example, a dose can be formulated in animal models to achieve a circulating concentration range that can be used to more accurately determine useful doses in humans. For example, a dose can be formulated in animal models to achieve a circulating concentration range that includes the IC₅₀ as determined in cell culture. Such information can be used to more accurately determine useful doses in humans.

Toxicity and therapeutic efficacy of such compounds can be determined by standard pharmaceutical procedures in cell cultures or experimental animals, *e.g.,* for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. See, *e.g.*, Fing1 et al., 1975, in "The Pharmacological Basis of Therapeutics", Ch. 1 p.1. Dosage amount and interval may be adjusted individually to provide plasma levels of the active moiety which are sufficient to maintain the desired effects, or minimal effective concentration (MEC). The MEC will vary for each compound but can be estimated from *in vitro* data. Dosages necessary to achieve the MEC will depend on individual characteristics and route of administration. However, HPLC assays or bioassays can be used to determine plasma concentrations.

Dosage intervals can also be determined using MEC value. Compounds should be administered using a regimen which maintains plasma levels above the MEC for 10-90% of the time, preferably between 30-90% and most preferably between 50-90%. In cases of local administration or selective uptake, the effective local concentration of the drug may not be related to plasma concentration.

An exemplary dosage regimen for agents, polypeptides, or other compositions of the invention will be in the range of about 0.01 to 100 mg/kg of body weight daily, with the preferred dose being about 0.1 to 25 mg/kg of patient body weight daily, varying in adults and children. Dosing may be once daily, or equivalent doses may be delivered at longer or shorter intervals.

The amount of composition administered will, of course, be dependent on the subject being treated, on the subject's weight, the severity of the affliction, the manner of administration and the judgment of the prescribing physician.

It will be clear that the invention may be practiced otherwise than as particularly described in the foregoing description and examples.

Numerous modifications and variations of the present invention are possible in light of the above teachings and, therefore, are within the scope of the invention. The entire disclosure of all publications cited herein are hereby incorporated by reference.

## Claims

1. A method for quantifying the activity of a protein mannosyl transferase, comprising the steps of:
(a) contacting protein mannosyl transferase (PMT), dolichyl phosphoryl mannose, and an acceptor peptide, under conditions suitable for PMT activity, wherein the dolichyl phosphoryl mannose is incorporated into unilamellar phospholipid vesicles; and
(b) determining the amount of glycosylated acceptor peptide produced.

2. The method of claim 1, wherein step (a) further comprises separating the glycosylated acceptor peptide from unreacted dolichyl phosphoryl mannose.

3. The method of claim 2, wherein the separating comprises liquid chromatography.

4. The method of claim 3, wherein the separating comprises combining the mixture produced in step (a) with acidified water, loading the resulting mixture onto a liquid chromatography column, and eluting the glycosylated acceptor peptide with an organic solvent.

5. The method of claim 4, wherein the organic solvent is selected from acetonitrile and alcohols.

6. The method of claim 5, wherein the organic solvent comprises acetonitrile.

7. The method of any of claims 3 to 6, wherein the chromatographic substrate is a reverse-phase matrix.

8. The method of claim 7, wherein the reserve-phase matrix is a C₁₈ matrix.

9. The method of any preceding claim, wherein the vesicles comprise a phospholipid selected from phosphocholine, phosphatidylserine, phosphatidyl-ethanolamine, and phosphatidylinositol.

10. The method of claim 9, wherein the vesicles comprise 1-palmitoyl-2-oleoyl-sn-glycero-3-phosphocholine.

11. The method of any preceding claim, wherein the acceptor peptide is selected from YNPTSV (SEQ ID NO: 1); YPTAV (SEQ ID NO: 2); PTV (SEQ ID NO: 3); PYTV (SEQ ID NO: 4); YPTAV (SEQ ID NO: 5); YNPTAV (SEQ ID NO: 6); YNLTSV (SEQ ID NO: 7); YNPASV (SEQ ID NO: 8); YNLTSV (SEQ ID NO: 9); YDLTSV (SEQ ID NO: 10); YQLTSV (SEQ ID NO: 11); PPASTSAPG (SEQ ID NO: 12); PPDAATAAPL (SEQ ID NO: 13); PPDAASAAPL (SEQ ID NO: 14); PPASTSAPG (SEQ ID NO: 15); PPASSSAPG (SEQ ID NO: 16); and VVPTVVPG (SEQ ID NO: 17).

12. The method of claim 11, wherein the acceptor peptide comprises YNPTSV (SEQ ID NO: 1).

13. The method of any preceding claim, wherein the dolichyl phosphoryl mannose comprises labelled mannose.

14. The method of claim 13, wherein step (b) comprises measuring labelled mannose bound to acceptor peptide.

15. A method for identifying an agent that inhibits the activity of protein mannosyl transferase, comprising conducting step (a) of a method of any preceding claim, in the presence of and absence of a test compound; and conducting step (b) of the method in order to measure PMT-mediated transfer of mannose from the dolichyl phosphoryl mannose to the acceptor peptide in the presence and absence of the test compound, wherein reduced transfer of mannose in the presence of the test compound identifies the agent.

16. The method according to claim 15, further comprising contacting a fungal organism with the agent.

17. The method according to claim 16, further comprising determining the effect of the agent on the growth of the fungal organism.

18. The method of any of claims 15 to 17, further comprising making a medicament comprising the agent.

19. Use of an agent determined by the method of any of claims 15 to 17, for the manufacture of a medicament for treating fungal infection.


SEQUENCE LISTING

<110> Pharmacia & Upjohn Company

<120> HIGH-EFFICIENCY ASSAY FOR PROTEIN MANNOSYL TRANSFERASES

<130> REP07106EP

<140> not yet known
<141> 2002-05-14

<160> 17

<170> PatentIn Ver. 2.1

<210> 1
<211> 6
<212> PRT
<213> Artificial Sequence

<220>
<223> Description of Artificial Sequence: synthetic
      peptide

<400> 1
Tyr Asn Pro Thr Ser Val
  1               5

<210> 2
<211> 5
<212> PRT
<213> Artificial Sequence

<220>
<223> Description of Artificial Sequence: synthetic
      peptide

<400> 2
Tyr Pro Thr Ala Val
  1               5

<210> 3
<211> 3
<212> PRT

<213> Artificial Sequence

<220>
<223> Description of Artificial Sequence: synthetic
      peptide

<400> 3
Pro Thr Val
  1

<210> 4
<211> 4
<212> PRT
<213> Artificial Sequence

<220>
<223> Description of Artificial Sequence: synthetic
      peptide

<400> 4
Pro Tyr Thr Val
  1

<210> 5
<211> 5
<212> PRT
<213> Artificial Sequence

<220>
<223> Description of Artificial Sequence: synthetic
      peptide

<400> 5
Tyr Pro Thr Ala Val
  1               5

<210> 6
<211> 6
<212> PRT
<213> Artificial Sequence

<220>

<223> Description of Artificial Sequence: synthetic
      peptide

<400> 6
Tyr Asn Pro Thr Ala Val
 1               5

<210> 7
<211> 6
<212> PRT
<213> Artificial Sequence

<220>
<223> Description of Artificial Sequence: synthetic
      peptide

<400> 7
Tyr Asn Leu Thr Ser Val
 1               5

<210> 8
<211> 6
<212> PRT
<213> Artificial Sequence

<220>
<223> Description of Artificial Sequence: synthetic
      peptide

<400> 8
Tyr Asn Pro Ala Ser Val
 1               5

<210> 9
<211> 6
<212> PRT
<213> Artificial Sequence

<220>
<223> Description of Artificial Sequence: synthetic
      peptide

<400> 9
Tyr Asn Leu Thr Ser Val
1               5

<210> 10
<211> 6
<212> PRT
<213> Artificial Sequence

<220>
<223> Description of Artificial Sequence: synthetic
      peptide

<400> 10
Tyr Asp Leu Thr Ser Val
1               5

<210> 11
<211> 6
<212> PRT
<213> Artificial Sequence

<220>
<223> Description of Artificial Sequence: synthetic
      peptide

<400> 11
Tyr Gln Leu Thr Ser Val
1               5

<210> 12
<211> 9
<212> PRT
<213> Artificial Sequence

<220>
<223> Description of Artificial Sequence: synthetic
      peptide

<400> 12
Pro Pro Ala Ser Thr Ser Ala Pro Gly
1               5

<210> 13
<211> 10
<212> PRT
<213> Artificial Sequence

<220>
<223> Description of Artificial Sequence: synthetic
      peptide

<400> 13
Pro Pro Asp Ala Ala Thr Ala Ala Pro Leu
 1               5                   10

<210> 14
<211> 10
<212> PRT
<213> Artificial Sequence

<220>
<223> Description of Artificial Sequence: synthetic
      peptide

<400> 14
Pro Pro Asp Ala Ala Ser Ala Ala Pro Leu
 1               5                   10

<210> 15
<211> 9
<212> PRT
<213> Artificial Sequence

<220>
<223> Description of Artificial Sequence: synthetic
      peptide

<400> 15
Pro Pro Ala Ser Thr Ser Ala Pro Gly
 1               5

<210> 16
<211> 9
<212> PRT
<213> Artificial Sequence

<220>
<223> Description of Artificial Sequence: synthetic
      peptide

<400> 16
Pro Pro Ala Ser Ser Ser Ala Pro Gly
  1               5

<210> 17
<211> 8
<212> PRT
<213> Artificial Sequence

<220>
<223> Description of Artificial Sequence: synthetic
      peptide

<400> 17
Val Val Pro Thr Val Val Pro Gly
  1               5